# EUROPEAN PATENT APPLICATION

(11) **EP 4 481 053 A1**
(43) Date of publication of application: **25.12.2024**
(21) Application number: 23756349.9
(22) Date of filing: 14.02.2023
(51) Int. Cl.: C12Q 1/04, C12N 15/12, C12Q 1/686, C12Q 1/6869, C12Q 1/6874

(54) **METHOD FOR DETERMINING WHETHER ASC IS INCLUDED AT HIGH PURITY IN ADIPOSE TISSUE-DERIVED CELL POPULATION**

(30) Priority: 15.02.2022 JP 2022021271
(71) Applicant: Keio University, Tokyo, 108-8345 (JP)
(72) Inventor: URUGA, Yukako, Tokyo 160-8582 (JP); MATSUBARA, Yumiko, Tokyo 160-8582 (JP)
(74) Representative: Mathys & Squire
(86) International application number: PCT/JP2023/004919
(87) International publication number: WO 2023/157824

(57) **Abstract**

An object of the present invention is to provide a method capable of more conveniently determining at an earlier stage whether a cell population collected from a vertebrate animal (donor, etc.) adipose tissue comprises mesenchymal stem cells (ASCs) at high purity, a kit for determining it, etc. The present invention includes, for example, a method for determining whether a cell population derived from an adipose tissue collected from a vertebrate animal comprises mesenchymal stem cells (ASCs) at high purity, the method comprising step X of detecting expression of one or more genes selected from predetermined gene group A in the cell population.

## Description

### Technical Field

The present invention relates to a method for more conveniently determining at an earlier stage whether a cell population derived from a vertebrate animal adipose tissue comprises ASCs at high purity, a kit for determination, etc.

### Background Art

Adipose-derived mesenchymal stem cells (also referred to as "ASCs" in the present specification) collected from a vertebrate animal adipose tissue have the ability to differentiate into osteoblasts, adipocytes, myocytes, chondrocytes, skin cells, platelet, etc. and also additionally have anti-inflammatory action, immunomodulatory action, and the like. Hence, not only are ASCs used in treatment of knee osteoarthritis but their development are underway for treatment of various diseases. For example, for the treatment of knee osteoarthritis, ASCs are collected from an adipose tissue of a patient and administered to the knee.

The present inventors have previously developed a method for producing platelet from mesenchymal cells such as adipose tissue-derived mesenchymal stem cells (patent document 1), and a novel method for producing a mesenchymal stem cell line derived from an adipose tissue (adipose-derived mesenchymal stem cell line: ASCL) from vertebrate animal adipose tissue-derived mesenchymal stem cells or the like (i.e., a novel cell line establishing method) (patent document 2). The cell line establishing method described in patent document 2 comprises the following steps (A) and (B) :
(A) inducing one or more cells selected from a stromal vascular fraction of a vertebrate animal adipose tissue comprising a mesenchymal stem cell, an adipose progenitor cell, and a stromal cell to differentiate into a mature adipocyte; and
(B) inducing the mature adipocyte obtained in step (A) to dedifferentiate to obtain a mesenchymal cell line derived from the vertebrate animal adipose tissue;

### Prior Art Documents

### Patent Documents

Patent Document 1: Domestic re-publication No. 2014/208100 of PCT international application
Patent Document 2: Domestic re-publication No. 2017/094260 of PCT international application

### Summary of the Invention

### Object to be Solved by the Invention

In the case of administering ASCs or cells obtained by differentiation thereof to a patient, it is preferable to use patient's own ASCs or cells derived therefrom in light of circumventing a rejection reaction.

Step A in a cell line establishing method described in patent document 2 specifically includes, for example, obtaining a cell population from an adipose tissue collected from a donor, removing mature adipocytes from the cell population, then performing initial culture and subsequent expansion culture of the cell population, and then inducing the cell population to differentiate into mature adipocytes. The present inventors have attempted to produce ASCL from cell populations derived from adipose tissues of various patients using the cell line establishing method described in patent document 2, and consequently found that the property of a cell population derived from an adipose tissue differs largely among donors; and even if the above cell population is cultured in a medium capable of inducing differentiation into mature adipocytes, cells that have differentiated into mature adipocytes may be insufficiently obtained, that is, the cell population may not comprise ASCs at high purity.

Thus, whether a cell population derived from an adipose tissue comprises ASCs at high purity only becomes evident after obtaining a cell population from an adipose tissue collected from a donor, removing mature adipocytes from the cell population, then performing initial culture and subsequent expansion culture of the cell population, then culturing the cell population in a medium capable of inducing differentiation into mature adipocytes, and confirming whether to be rich in mature adipocytes. If a cell population derived from an adipose tissue does not comprise ASCs at high purity, the problem is that cost, time and labor, and period required for expansion culture, differentiation inducing culture, or the like are wasted because the production of ASCL is discontinued.

An object of the present invention is to provide a method capable of more conveniently determining at an earlier stage whether a cell population collected from a vertebrate animal (donor, etc.) adipose tissue comprises mesenchymal stem cells (ASCs) at high purity, a kit for determining it, etc.

### Means to Solve the Object

The present inventors conducted extensive studies to solve the above problems and compared gene expression between a cell population comprising ASCs at high purity and a cell population not comprising ASCs at high purity. As a result, the present inventors found that when expression of one or more genes selected from the gene group A described later or increased expression thereof is detected in a cell population at a stage after initial culture, it can be determined that the cell population comprises ASCs at high purity, whereby the present invention was accomplished.

More specifically, the present invention relates to:
[1] a method for determining whether a cell population derived from an adipose tissue collected from a vertebrate animal comprises mesenchymal stem cells (ASCs) at high purity, the method comprising step X of detecting expression of one or more genes selected from gene group A described later in the cell population;
[2] the method according to the above [1], wherein when the expression of one or more genes selected from the gene group A or increased expression thereof is detected in step X, it can be determined the cell population in step X comprises mesenchymal stem cells (ASCs) at high purity;
[3] the method according to the above [1] or [2], wherein the cell population derived from an adipose tissue is a primary cultured cell population;
[4] the method according to any one of the above [1] to [3], wherein the detection of the expression of one or more genes in step X is a detection using a gene microarray, PCR, or sequencing;
[5] a kit for determining whether a cell population derived from a vertebrate animal adipose tissue comprises mesenchymal stem cells (ASCs), the kit comprising a primer and/or a probe for detecting expression of mRNA or cDNA of one or more genes selected from gene group A descried later, or a labeled form thereof;
[6] a biomarker for determining whether a cell population derived from a vertebrate animal adipose tissue comprises mesenchymal stem cells (ASCs) at high purity, the biomarker consisting of one or more genes selected from gene group A described later; etc.

### Effect of the Invention

The present invention can provide a method capable of more conveniently determining at an earlier stage whether a cell population collected from a vertebrate animal (donor, etc.) adipose tissue comprises mesenchymal stem cells (ASCs) at high purity, a kit for determining it, etc. The present invention is preferable because cost, time and labor, and wasted period can be reduced by determining earlier whether a cell population collected from a vertebrate animal (donor, etc.) adipose tissue comprises ASCs capable of producing ASCL at high purity in producing ASCL from the cell population using a cell line establishing method described in patent document 2.

### Brief Description of Drawing

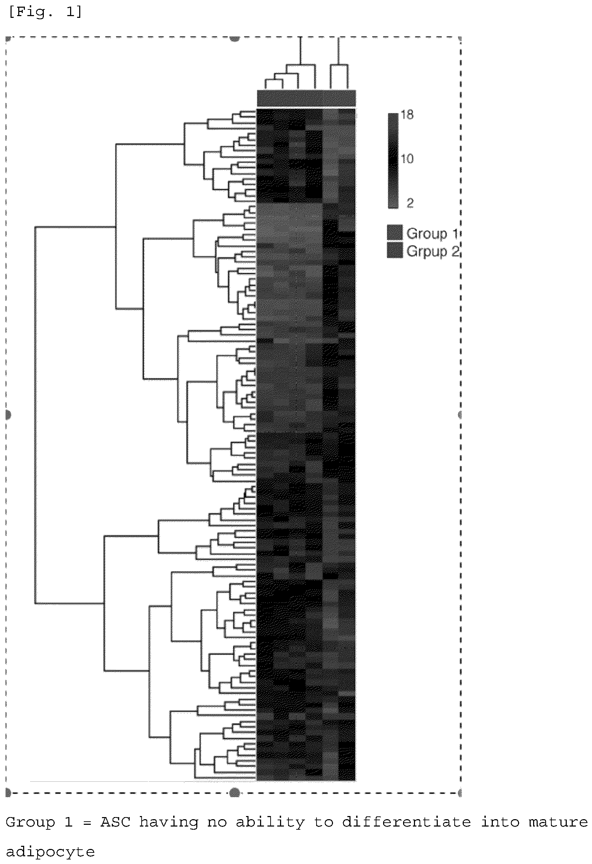

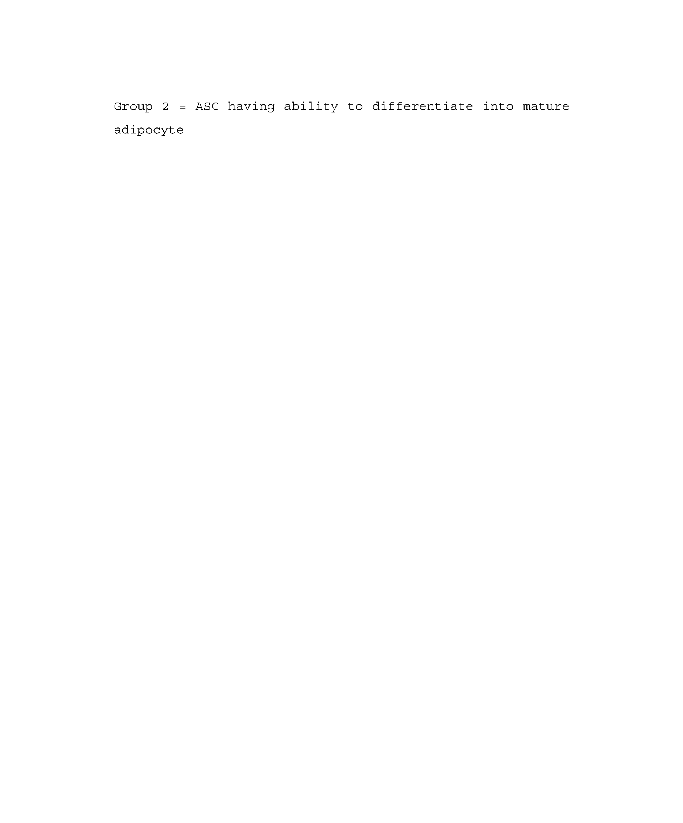

[Figure 1] Figure 1 is a diagram showing results of microarray analysis in Example 1 described later.

### Mode of Carrying Out the Invention

The present invention includes an embodiment of
[1] a method for determining whether a cell population derived from an adipose tissue collected from a vertebrate animal comprises mesenchymal stem cells (ASCs) at high purity, the method comprising step X of detecting expression of one or more genes selected from the following gene group A in the cell population (hereinafter, also referred to as the "determination method of the present invention"):
   gene group A:
   SFRP2 (secreted frizzled-related protein 2) gene,
   PTGIS (prostaglandin I2 (prostacyclin) synthase) gene,
   PRG4 (proteoglycan 4) gene,
   MFAP5 (microfibrillar associated protein 5) gene,
   CRABP2 (cellular retinoic acid binding protein 2) gene, HAS1 (hyaluronan synthase 1) gene,
   CXCL1 (chemokine (C-X-C motif) ligand 1 (melanoma growth stimulating activity, alpha)) gene,
   CXCL5 (chemokine (C-X-C motif) ligand 5) gene,
   CRLF1 (cytokine receptor-like factor 1) gene,
   SPON1 (spondin 1, extracellular matrix protein) gene,
   CXCL6 (chemokine (C-X-C motif) ligand 6) gene,
   S100A16 (S100 calcium binding protein A16) gene,
   DPT (dermatopontin) gene,
   MASP1 (mannan-binding lectin serine peptidase 1 (C4/C2 activating component of Ra-reactive factor)) gene,
   CXCL8 (chemokine (C-X-C motif) ligand 8) gene,
   ACKR3 (atypical chemokine receptor 3) gene,
   GFPT2 (glutamine-fructose-6-phosphate transaminase 2) gene,
   IL1RN (interleukin 1 receptor antagonist) gene,
   PDE4D (phosphodiesterase 4D, cAMP-specific) gene,
   FBLN2 (fibulin 2) gene,
   CHI3L1 (chitinase 3-like 1 (cartilage glycoprotein-39) gene,
   NR4A1 (nuclear receptor subfamily 4, group A, member 1) gene,
   PID1 (phosphotyrosine interaction domain containing 1) gene,
   ACKR4 (atypical chemokine receptor 4) gene,
   IL6 (interleukin 6) gene,
   ID3 (inhibitor of DNA binding 3, dominant negative helix-loop-helix protein) gene,
   PTGFRN (prostaglandin F2 receptor inhibitor) gene,
   EPB41L3 (erythrocyte membrane protein band 4.1-like 3) gene,
   PENK (proenkephalin) gene,
   ID1 (inhibitor of DNA binding 1, dominant negative helix-loop-helix protein) gene,
   HMCN1 (hemicentin 1) gene,
   HS3ST3B1 (heparan sulfate (glucosamine) 3-0-sulfotransferase 3B1) gene,
   PDPN (podoplanin) gene,
   HES1 (hes family bHLH transcription factor 1) gene,
   NDNF (neuron-derived neurotrophic factor) gene,
   KIAA1549L (KIAA1549-like) gene,
   LRRC15 (leucine rich repeat containing 15) gene,
   DNASE1L3 (deoxyribonuclease I-like 3) gene,
   GAS7 (growth arrest-specific 7) gene,
   FNDC1 (fibronectin type III domain containing 1) gene,
   TWIST2 (twist family bHLH transcription factor 2) gene,
   IRAK3 (interleukin 1 receptor associated kinase 3) gene,
   SYNPO (synaptopodin) gene,
   CBS (cystathionine-beta-synthase) gene,
   MFAP2 (microfibrillar associated protein 2) gene,
   BMP4 (bone morphogenetic protein 4) gene,
   MXRA5 (matrix-remodelling associated 5) gene,
   MMP12 (matrix metallopeptidase 12) gene,
   CPXM1 (carboxypeptidase X (M14 family), member 1) gene,
   SGCD (sarcoglycan delta) gene,
   SIPA1L3 (signal-induced proliferation-associated 1 like 3) gene,
   TBX5 (T-box 5) gene,
   PTPRN (protein tyrosine phosphatase, receptor type, N) gene,
   JAM2 (junctional adhesion molecule 2) gene,
   ID2 (inhibitor of DNA binding 2, dominant negative helix-loop-helix protein) gene, and
   ITPR3 (inositol 1,4,5-trisphosphate receptor, type 3) gene;
[2] a kit for determining whether a cell population derived from a vertebrate animal adipose tissue comprises mesenchymal stem cells (ASCs) at high purity, the kit comprising a primer and/or a probe for detecting expression of mRNA or cDNA of one or more genes selected from the gene group A, or a labeled form thereof (hereinafter, also referred to as the "kit of the present invention");
[3] a biomarker for determining whether a cell population derived from a vertebrate animal adipose tissue comprises mesenchymal stem cells (ASCs) at high purity, the biomarker consisting of one or more genes selected from the gene group A (hereinafter, also referred to as the "biomarker of the present invention"); etc.

### (Determination method of present invention)

The determination method of the present invention is not particularly limited as long as it is a method for determining whether a cell population collected from a vertebrate animal comprises mesenchymal stem cells (ASCs) at high purity, the method comprising step X of detecting expression of one or more genes selected from the above gene group A in the cell population.

The "vertebrate animal" as used herein is not particularly limited as long as it is a vertebrate animal, and examples include a mammal, a bird, a reptile, an amphibian, and a fish, of which a mammal such as a human, a mouse, a rat, a guinea pig, a rabbit, a cat, a dog, a horse, a cow, a monkey, a sheep, a goat, and a pig being preferable, of which a human being particularly preferable.

The "adipose tissue" as used herein is not particularly limited as long as a tissue comprises fats and examples include a subcutaneous adipose tissue, an adipose tissue in the bone marrow, and a visceral adipose tissue, with a subcutaneous adipose tissue being preferable in light of comparatively low invasiveness to a vertebrate animal supplying the adipose tissue and being comparatively easily collectable.

The "adipose tissue-derived mesenchymal stem cells" (ASCs) as used herein are mesenchymal stem cells contained in an adipose tissue collected from a vertebrate animal and mean cells having differentiation potency into mature adipocytes. The mesenchymal stem cells as used herein also include, for the sake of convenience, adipose tissue-derived adipose progenitor cells. The ASCs as used herein may consist of only mesenchymal stem cells, may consist of adipose progenitor cells, or may consist of mesenchymal stem cells and adipose progenitor cells.

The "cell population derived from an adipose tissue" (hereinafter, referred to as the "cell population according to the present invention") as used herein is not particularly limited as long as it is a cell population derived from an adipose tissue. Preferable examples thereof include a cell population of a stromal vascular fraction.

The "stromal vascular fraction" as used herein means the cells other than mature adipocytes among the cells of a vertebrate animal adipose tissue. Examples of the "cell population of a stromal vascular fraction" as used herein include a cell population comprising one or more cells selected from the group consisting of a mesenchymal stem cell, a stromal cell, a vascular endothelial cell, a cell related to blood, a smooth muscle cell, and a fibroblast, preferably include a cell population consisting of one or more cells selected from the group consisting of a mesenchymal stem cell, a stromal cell, a vascular endothelial cell, a cell related to blood, a smooth muscle cell, and a fibroblast, and more preferably include a cell population comprising at least mesenchymal stem cells and comprising one or more cells selected from the group consisting of a stromal cell, a vascular endothelial cell, a cell related to blood, a smooth muscle cell, and a fibroblast. The "stromal vascular fraction" can be obtained by removing mature adipocytes from a cell population obtained by treating a vertebrate animal adipose tissue with an enzyme capable of dispersing the vertebrate animal adipose tissue cells.

Examples of the preferable embodiment of the above "stromal vascular fraction" include a "cell population of a stromal vascular fraction" obtained by dispersing cells of a vertebrate animal adipose tissue, of which a cell population obtained by removing mature adipocytes from a cell population obtained by treating the vertebrate animal adipose tissue with an enzyme capable of dispersing the vertebrate animal adipose tissue cells (cell population A) being preferable. A cell population obtained by further removing one or more (preferably all of four) cells selected from the group consisting of a vascular endothelial cell, a cell related to blood, a smooth muscle cell, and a fibroblast from the cell population A may also be used.

Examples of the above "treating a vertebrate animal adipose tissue with an enzyme capable of dispersing vertebrate animal adipose tissue cells" include a method in which a vertebrate animal adipose tissue is immersed in a solution comprising such an enzyme and incubated, for example, for about 30 minutes to 3 hours.

The above "enzyme capable of dispersing vertebrate animal adipose tissue cells" is not particularly limited as long as it can disperse cells of a vertebrate animal adipose tissue when allowed to act on the vertebrate animal adipose tissue, and examples include one or more enzymes selected from the group consisting of collagenase, trypsin, caseinase, clostripain, trypsin-EDTA, dispase, thermolysin, pronase, hyaluronidase, pancreatin, elastase, and papain, of which at least one or more enzymes selected from the group consisting of collagenase, trypsin, caseinase, and clostripain being preferable, and commercial collagenase (type I) and collagenase (type II) being more preferable, with collagenase (type II) being further preferable. Further, the above "enzyme capable of dispersing vertebrate animal adipose tissue cells" preferably comprises at least collagenase.

The above "removing mature adipocytes from a cell population obtained by treating a vertebrate animal adipose tissue with an enzyme capable of dispersing the vertebrate animal adipose tissue cells" is not particularly limited as long as a method can remove mature adipocytes from such a cell population, but examples preferably include a method of recovering a cell population (cell pellet) which is precipitated by centrifugation of a suspension comprising the above cell population. Mature adipocytes comprise a large amount of fats, thus have a light specific gravity and float in the upper part of supernatant when centrifuged, hence the recovery of a cell pellet precipitated by the centrifugation enables the removal of mature adipocytes. Further, the method for removing vascular endothelial cells, smooth muscle cells, and fibroblasts from the cell population obtained by treating a vertebrate animal adipose tissue with an enzyme capable of dispersing vertebrate animal adipose tissue cells is not particularly limited as long as a method can remove these cells from such a cell population and examples include a method for removing vascular endothelial cells from the cell population when CD31 known as a surface marker of the vascular endothelial cell selects negative cells (or CD31 removes positive cells), and examples of the method for removing cells related to blood include a method for removing cells related to blood from the cell population by selecting CD45- (a surface marker of hematopoietic cells other than red blood cell and platelet) negative and Ter119- (a surface marker of red blood cell and progenitor cell thereof) negative cells (or CD45-positive and Ter119-positive cells are removed). Additionally, when 7-amino-actinomycin D (7-AAD), which is not a surface marker, being negative is used as an indicator, it is preferable because dead cells comprised in a vertebrate animal adipose tissue can be excluded. 7-AAD intercalates with a DNA chain of a dead cell and produces red fluorescence at a 488-nm excitation light.

In the above step X, a cell population obtained from a vertebrate animal adipose tissue may be used directly as the cell population derived from an adipose tissue (cell population according to the present invention), and it is preferable to use a cell population obtained by culture (that is, primary culture) of the cell population obtained from a vertebrate animal adipose tissue.

Examples of the method for the above primary culture include a general culture method for proliferating mesenchymal stem cells, for example, a method of culturing the cell population according to the present invention in basal medium for mesenchymal cell culture.

Examples of the conditions for culturing the cell population according to the present invention in basal medium for mesenchymal cell culture include a method of adhesion culture in a culture vessel coated with the extracellular matrix, and examples of the culture temperature include typically a range from 12 to 45°C, preferably a range from 15 to 37°C, and examples of the culture period include, a range from 1 to 40 days, and preferably a range from 1 to 21 days. In the culture, the cell population according to the present invention may or may not be subcultured. Further, examples of the above extracellular matrix include at least one or more components selected from collagen, fibronectin, proteoglycan, and laminin, and BD Matrigel (registered trademark) (manufactured by BD Biosciences) comprising these components can also be used.

The "basal medium for mesenchymal cell culture" in the present Description is not particularly limited as long as medium can culture ASCs therein and proliferate the ASCs, but in light of easy preparation and preventing lot-to-lot variation, a chemically synthesized medium is preferable, and the medium preferably comprises one or more saccharide(s), one or more inorganic salt(s), one or more amino acid(s), and one or more vitamin(s), and one or more other components as needed.

Examples of the above saccharide specifically include a monosaccharide such as glucose, mannose, fructose and galactose, and a disaccharide such as sucrose, maltose, and lactose, of which glucose being particularly preferable, and one or more of these saccharides can be added in combination.

Examples of the above inorganic salt specifically include one or more inorganic salt(s) such as calcium chloride, calcium nitrate, a copper sulfate pentahydrate, an iron(III) nitrate nonahydrate, an iron (II) sulfate heptahydrate, a magnesium chloride hexahydrate, magnesium sulfate, potassium chloride, sodium chloride, sodium bicarbonate, disodium hydrogen phosphate, a disodium hydrogenphosphate dihydrate, sodium dihydrogen phosphate, a sodium dihydrogen phosphate monohydrate, a sodium dihydrogen phosphate dihydrate, a sodium selenite pentahydrate, and a zinc sulfate heptahydrate.

Examples of the above amino acids specifically include one or more amino acid(s) selected from alanine, arginine, asparagine, aspartic acid, cystine, cysteine, glutamine, glycine, histidine, glutamic acid, hydroxyproline, isoleucine, leucine, lysine, methionine, phenylalanine, proline, serine, threonine, tryptophan, tyrosine, and valine, and preferably include an L-form amino acid and a derivative thereof and a by-product such as a salt thereof and a hydrate thereof. Examples of the above arginine include an arginine by-product such as an L-arginine hydrochloride and an L-arginine monohydrochloride, examples of the above aspartic acid include an aspartic acid by-product such as an L-sodium aspartate monohydrate, an L-aspartic acid monohydrate, potassium L-aspartate, and magnesium L-aspartate, examples of the above cysteine include a cysteine by-product such as L-cysteine dihydrochloride and an L-cysteine hydrochloride monohydrate, and a lysine by-product such as L-lysine hydrochloride, examples of the above glutamic acid include a glutamine by-product such as monosodium L-glutamate, examples of the above asparagine include an asparagine by-product such as an L-asparagine monohydrate, examples of the above tyrosine include a tyrosine by-product such as an L-tyrosine disodium dihydrate, examples of the above histidine include a histidine by-product such as histidine hydrochloride and a histidine hydrochloride monohydrate, and examples of the above lysine include a lysine by-product such as L-lysine hydrochloride.

Examples of the above vitamins specifically include one or more vitamin(s) selected from biotin, choline, folic acid, inositol, niacin, pantothenic acid, pyridoxine, riboflavin, thiamine, vitamin B12, paraaminobenzoic acid (PABA), and ascorbic acid, and a derivative each thereof and a by-product thereof such as a salt thereof and a hydrate thereof. Examples of the above choline include a choline by-product such as choline chloride, examples of the niacin include a niacin by-product such as nicotinic acid, nicotinamide, and nicotinic alcohol, examples of the pantothenic acid include a pantothenic acid by-product such as calcium pantothenate, sodium pantothenate, and panthenol, examples of the pyridoxine include a pyridoxine by-product such as pyridoxine hydrochloride, pyridoxal hydrochloride, pyridoxal phosphate, and pyridoxamine, examples of the thiamine include a thiamine by-product such as thiamine hydrochloride, thiamine nitrate, bisthiamine nitrate, a thiamine dicetyl sulfate ester salt, fursultiamine hydrochloride, octothiamine, and benfotiamine, examples of the ascorbic acid include an ascorbic acid by-product such as ascorbic acid 2-phosphate, ascorbic acid magnesium phosphate, sodium ascorbate sulfate, aminopropyl ascorbyl phosphate, and sodium ascorbate phosphate.

Examples of the above other components include a buffer such as HEPES, an antibiotic such as penicillin and streptomycin, pyruvic acid and a derivative thereof and a by-product thereof such as a salt thereof and a hydrate thereof, and phenol red, examples of the above by-product of antibiotics include penicillin G sodium and streptomycin sulfate, or preferably a penicillin-streptomycin solution, and examples of the pyruvic acid by-product preferably include sodium pyruvate.

Specific examples of the above basal medium for mesenchymal cell culture include a known commercial chemically synthesized medium such as Dulbecco's modified Eagle's medium (DMEM), Iscove's Modified Dulbecco's Medium (IMDM), RPMI 1640 medium, minimum essential medium (MEM), basal medium of Eagle (BME), and F12 medium, a medium of 2 or more of these medium mixed in a suitable ratio such as DMEM/F12 (medium of DMEM and F12 medium mixed in 1:1), with medium to which one or more substances selected from the group consisting of antibiotics such as penicillin and streptomycin; and additional amino acids (preferably non-essential amino acids); are added being preferable, and medium wherein an antibiotic (preferably penicillin G sodium, streptomycin sulfate or a penicillin-streptomycin solution) is further added to DMEM, IMDM or RPMI 1640 medium being particularly more preferable, of which medium wherein an antibiotic (preferably penicillin G sodium, streptomycin sulfate, or a penicillin-streptomycin solution) is further added to DMEM being particularly preferable.

Examples of the particularly preferable basal medium for mesenchymal cell culture in the present invention include medium wherein 100 U/mL (final concentration) of a penicillin-streptomycin solution is added to DMEM having the composition to be described later (hereinafter referred to as "particularly preferable basal medium in the present invention"), and medium comprising each component in a concentration of the proportion ranging independently from 70% to 130% to the concentration of each component in the particularly preferable basal medium in the present invention.

### (Composition of DMEM)

200 mg/L of anhydrous calcium chloride, 0.1 mg/L of Fe(NO₃)₃•9H₂O, 200 mg/L of potassium chloride, 97.67 mg/L of anhydrous magnesium sulfate, 6400 mg/L of sodium chloride, 3700 mg/L of sodium bicarbonate, 125 mg/L of sodium dihydrogen phosphate monohydrate, 4500 mg/L of D-glucose, 15 mg/L of phenol red, 110 mg/L of sodium pyruvate, 84 mg/L of L-arginine hydrochloride, 63 mg/L of L-cysteine dihydrochloride, 584 mg/L of L-glutamine, 30 mg/L of glycine, 42 mg/L of L-histidine hydrochloride monohydrate, 105 mg/L of L-isoleucine, 105 mg/L of L-leucine, 146 mg/L of L-lysine hydrochloride, 30 mg/L of L-methionine, 66 mg/L of L-phenylalanine, 42 mg/L of L-serine, 95 mg/L of L-threonine, 16 mg/L of L-tryptophan, 104 mg/L of L-tyrosine disodium dihydrate, 94 mg/L of L-valine, 4 mg/L of D-calcium pantothenate, 4 mg/L of choline chloride, 4 mg/L of folic acid, 7.2 mg/L of i-inositol, 4 mg/L of nicotinamide, 4 mg/L of pyridoxine hydrochloride, 0.4 mg/L of rivoflavin, 4 mg/L of thiamine hydrochloride.

In the above step X, the method for detecting expression of one or more genes selected from the gene group A in the cell population according to the present invention is not particularly limited as long as the method can detect expression of the genes. Examples thereof include a method of detecting expression of a nucleic acid (preferably mRNA or cDNA) of each of the genes and specifically include a detection method using a gene microarray, a detection method using PCR, and a detection method using sequencing. Known methods can be used as these detection methods.

Here, PCR will be described. In the case of using mRNA to be measured, the cell population according to the present invention is subjected, if necessary, to known pretreatments such as filtration, centrifugation, and chromatography. Then, RNA can be extracted from the specimen cells (that is, cells of the cell population according to the present invention) using a general-purpose method such as a guanidine-cesium chloride ultracentrifugation method, an acidic guanidine-phenol chloroform method (AGPC method), a magnetic beads method, or a silica column method. Further, RNA may be extracted using a commercial kit (QIAGEN RNeasy KIt, TRIZOL, etc.).

In mRNA measurement, its level can be determined by, for example, (1) determining the amount of the amplification product obtained by PCR using a nucleic acid fragment capable of specifically hybridizing with the target mRNA and the RNA derived from the specimen cells; (2) determining the hybridization efficiency between a nucleic acid fragment capable of specifically hybridizing with the target mRNA and the RNA derived from the specimen cells; or (3) other known quantitation methods.

Here, in the case of using PCR, the "nucleic acid fragment capable of specifically hybridizing with the target mRNA" can be designed by comparing the nucleotide sequence of the target gene with the nucleotide sequence of another gene and selecting a sequence specific for mRNA of the target gene. Here, the nucleotide sequence of mRNA of the target gene can be obtained with reference to, for example, a database (GenBank, etc.). Alternatively, nucleotide sequences are aligned using software (Clustal X, etc.), and a specific sequence can be visually found, for example. The nucleic acid fragment is not particularly limited by its length and is preferably a nucleic acid fragment consisting of 5 to 50 nucleotides, and more preferably a nucleic acid fragment consisting of 18 to 25 consecutive nucleotides.

The nucleic acid fragment capable of hybridizing with mRNA of the target gene is not limited to the sequence thus designed and can be appropriately conceived based on known technical common sense. Examples of such a nucleic acid fragment that can be used include a nucleic acid fragment consisting of a nucleotide sequence complementary to the nucleotide sequence, or a homologous nucleotide sequence that can also be used for mRNA measurement of the target gene, for example, (a) a nucleotide sequence derived from the nucleotide sequence by substitution, addition, or deletion of 1 to 10, preferably 1 or several nucleotides; (b) a nucleotide sequence having 90% or higher, preferably 95% or higher, and more preferably 99% or higher identity to the nucleotide sequence; or (c) a nucleotide sequence that hybridizes under stringent conditions with DNA consisting of a nucleotide sequence complementary to the nucleotide sequence.

The nucleic acid fragment may be a nucleic acid fragment in which any number, preferably 100 or less, more preferably 20 or less, and further preferably 10 or less nucleotides are added to both or one of the ends thereof, preferably to the 5' end.

The nucleic acid fragment thus designed can be artificially synthesized, for example, using a DNA synthesizer according to the nucleotide sequence thereof. The fragment is preferably a fragment whose specificity has been confirmed after synthesis. Here, in the case of using, for example, the target mRNA as a template, the specificity can be confirmed by confirming that a specific PCR amplification product is obtained when compared with an appropriate control. Examples of the nucleic acid fragment include a DNA fragment and an RNA fragment.

Tables 1 and 2 described later show the accession No. of a typical nucleotide sequence of each gene in the gene group A.

The above "nucleic acid fragment capable of specifically hybridizing with the target mRNA" can be used as a primer or a probe for detecting expression of mRNA or cDNA of one or more genes selected from the gene group A.

Here, the identity between nucleotide sequences is calculated by use of a homology analysis program of GENETYX (registered trademark).

The "stringent conditions" mean that two DNA fragments hybridize with each other under standard hybridization conditions as described by Sambrook J. et al. (Expression of cloned genes in E. coli (Molecular Cloning: A laboratory manual (1989)), Cold Spring Harbor Laboratory Press, New York, USA, 9.47-9.62 and 11.45-11.61).

The mRNA of the specimen cells can be measured by PCR using the nucleic acid fragment thus produced and the RNA derived from the specimen cells, preferably real-time RT-PCR comprising step of producing cDNA from mRNA. Here, RT-PCR can be performed using a known method such as two-step RT-PCR or one-step RT-PCR. Particularly, one-step RT-PCR is preferable in light of being convenient and preventing cross-contamination. The one-step RT-PCR can be performed using, for example, a commercial kit (QIAGEN One-Step RT-PCR kit, etc.). Various reverse transcriptases such as M-MHV reverse transcriptase can be used as an enzyme having reverse transcription activity for use in RT reaction. DNA polymerase for use in PCR reaction for amplifying DNA, preferably has heat resistance against a temperature of 90°C or higher.

Such PCR reaction can be performed, for example, through one to several tens of cycles each having temperature conditions involving 90 to 98°C of thermal denaturation reaction of double-stranded DNA into single-stranded DNA, 37 to 72°C of annealing reaction for hybridizing primers to template cDNA, and 50 to 75°C of extension reaction for allowing DNA polymerase to act. One example of preferable reaction conditions includes thermal denaturation at 95°C for 30 seconds, annealing at 60°C for 30 seconds, and extension reaction at 72°C for 40 seconds. In PCR reaction, two types of primers are preferably used as one set. In this case, the two primers need to be combined as a sense strand and an antisense strand. The above nucleic acid fragment may be used as a probe and may be used in combination with other known universal primers, oligonucleotides, or the like.

The specimen sample containing mRNA serving as a template for this RT-PCR reaction preferably contains 1 pg to 1 µg of RNA, and more preferably 2 ng to 50 ng of RNA, in terms of the total amount of RNA.

When PCR reaction has appropriately proceeded, the "amount of the PCR amplification product", the "number of PCR cycles", and the "amount of the PCR template" usually correlate with each other. Thus, the mRNA level of the target gene, that is, the expression level of the target gene, can be determined by appropriate calculation in consideration of the amount of the amplification product obtained through the PCR reaction thus performed and the number of PCR cycles.

The determination of the amount of the PCR amplification product and the number of PCR cycles is not particularly limited and can be performed by any method, for example, by determining the number of PCR cycles when the DNA level has reached any predetermined level. This operation can be performed, for example, by using a "combinatory method of PCR in which a PCR product is labeled and PCR in which the label is measured over time", and determining the number of PCR cycles when fluorescence intensity has reached a predetermined level. Here, examples of the labeling include labeling with a fluorescent dye, and examples of the measurement of the label include measurement of fluorescence intensity. Here, examples of the labeling with a fluorescent dye include labeling with an intercalator fluorescent dye, and examples of the intercalator fluorescent dye include SYBR(R) Green I. The intercalator dye has the property of enhancing fluorescence intensity through intercalation with a double-stranded nucleic acid, and therefore emits fluorescence with intensity that reflects the PCR amplification product. The labeling with the fluorescent dye may be performed by use of TaqMan probe, Molecular Beacon, or the like labeled with the fluorescent dye. TaqMan probe or Molecular Beacon is a probe having a fluorescent dye and a quencher bound with an oligonucleotide having homology to an internal sequence of a region to be amplified by PCR. The probe is used so as to coexist in PCR reaction. Since fluorescence is emitted in response to the degree of PCR amplification reaction through the interaction between the fluorescent dye and the quencher bound with the probe, the PCR amplification product can be observed over time by measuring the fluorescence intensity at each PCR stage.

As described above, the mRNA level of the target gene in the specimen can also be determined from, for example, the hybridization efficiency between the nucleic acid fragment capable of specifically hybridizing with the target mRNA and the RNA derived from the specimen.

Here, the nucleic acid fragment capable of specifically hybridizing with the mRNA of the target gene can be designed and produced as described above for use. The nucleic acid fragment is preferably a labeled nucleic acid fragment. Here, examples of the label include an enzyme, a paramagnetic ion, biotin, a fluorescent dye, a chromophore, a heavy metal, and a radioisotope. A more preferable marker is an enzyme. Here, examples of the enzyme include horse radish peroxidase and alkaline phosphatase. The labeling can be performed by a known method. Provided that the degree of hybridization between a sample containing the RNA derived from the specimen and such a nucleic acid fragment is measured, the mRNA level of the target gene in the specimen can be determined by a known calculation method. The measurement of the degree of hybridization is not particularly limited and can be performed in accordance with a known method, for example, by measuring the label added to the nucleic acid fragment. That is, in the case of using a nucleic acid fragment labeled with a fluorescent dye, the fluorescence intensity can be measured therefor.

The expression level of the target gene may be measured using, as a probe, a nucleic acid fragment capable of specifically hybridizing with the nucleotide sequence of the target gene or mRNA or cDNA thereof. As for SFRP2 gene, a nucleic acid fragment consisting of a portion of the nucleotide sequence described in GenBank Accession No. NM_003013.3 or a nucleotide sequence complementary thereto, or a nucleic acid fragment that has a nucleotide sequence homologous thereto and is functionally equivalent to the above nucleic acid fragment can also be used as a probe. Such a probe may be immobilized on any solid phase and thereby used as a DNA chip, a gene chip, a cDNA microarray, an oligo DNA array, or the like.

In addition to the above listed probes, a plurality of nucleic acid fragments capable of specifically hybridizing with a plurality of regions appropriately selected from the nucleotide sequence of the target gene or mRNA thereof may be designed and used in combination as probes so as to specifically detect the nucleotide sequence of the target gene or mRNA thereof.

The solid phase for use in probe immobilization is not particularly limited as long as the solid phase can immobilize a polynucleotide. Examples thereof include a glass plate, a nylon membrane, microbeads, a silicon chip, and a capillary. The solid phase may be labeled. The label is not particularly limited, and examples thereof include a fluorescent dye and a radioisotope. A polynucleotide can be immobilized onto the solid phase by a method of placing a polynucleotide synthesized in advance on the solid phase or a method of synthesizing the target polynucleotide on the solid phase. The immobilization method, for example, for a DNA microarray can employ a commercial spotter or the like, and a known method (polynucleotide printing by an inkjet method, *in situ* synthesis, or photolithography) can be appropriately used depending on the type of the probe to be immobilized.

The expression level of the target gene can be measured by hybridizing the DNA chip or the like prepared by the above method with a labeled DNA or RNA prepared from RNA prepared from the cell population according to the present invention or a labeled DNA or RNA prepared directly from the cell population; and measuring, as a signal derived from the labeled form of the probe, the amount of the double strand of the formed probe and the labeled DNA or RNA. Here, the signal can be detected by a routine method, for example, by measurement using a radiation detector, a fluorescence detector, or the like.

In addition to the above method, the expression level of the target gene can be measured by a microbeads method. For example, the expression levels of a plurality of target genes may be simultaneously determined as follows: probes for mRNA derived from different target genes are immobilized on microbeads which labeled with different fluorescent agents and hybridized with the mRNA of the target genes prepared from a specimen such as cultured cells, a tissue, a tissue section, or a blood lysate, and each target gene is identified by detecting the fluorescence therefrom, while a labeled probe is hybridized with the target gene-derived mRNA hybridized with the probe immobilized on the microbead, followed by the detection of the label of the probe to thereby measure the mRNA level.

The copy number and the expression level of the target gene can be measured by use of a known method such as southern hybridization, northern hybridization, FISH, or CGH using the above probe.

The expression of the target gene in the cell population according to the present invention may be detected by using RNA sample prepared from the cell population according to the present invention, and sequencing the RNA sequence by a known method.

The "one or more genes selected from the gene group A" (target gene) as used herein is not particularly limited as long as the target gene is one or more genes selected from the gene group A. The target gene is preferably 3 or more, more preferably 5 or more, and further preferably 7 or more genes in light of obtaining higher determination accuracy. On the other hand, the target gene is, for example, 11 or less, preferably 9 or less, more preferably 7 or less, and further preferably 5 or less genes in light of convenience or cost. Specifically, for example, 1 to 11, 2 to 11, 1 to 9, 2 to 9, 1 to 7, 2 to 7, 3 to 7, 1 to 5, 2 to 5, or 3 to 5 genes are preferable in light of the balance between determination accuracy and convenience and cost.

The "one or more genes selected from the gene group A" as used herein have a relatively high ratio of the "expression level of each gene in the cell population comprising ASCs at high purity" to the "expression level of the gene in a cell population not comprising ASCs at high purity" (hereinafter, also referred to as the "ratio according to the present invention") and are thus more suitable for determining whether the cell population comprises ASCs at high purity. Hence, the one or more genes selected from the gene group A are preferably one or more genes selected from the group consisting of PRG4 gene, MFAP5 gene, CRABP2 gene, HAS1 gene, CXCL1 gene, CXCL5 gene, CRLF1 gene, SPON1 gene, CXCL6 gene, S100A16 gene, DPT gene, MASP1 gene, CXCL8 gene, PTGIS gene, ACKR3 gene, GFPT2 gene, IL1RN gene, PDE4D gene, FBLN2 gene, CHI3L1 gene, NR4A1 gene, PID1 gene, ACKR4 gene, IL6 gene, ID3 gene, PTGFRN gene, EPB41L3 gene, PENK gene, ID1 gene, SFRP2 gene, HMCN1 gene, HS3ST3B1 gene, PDPN gene, HES1 gene, NDNF gene, KIAA1549L gene, LRRC15 gene, DNASE1L3 gene, GAS7 gene, FNDC1 gene, TWIST2 gene, IRAK3 gene, SYNPO gene, and CBS gene,
of which preferable are one or more genes selected from the group consisting of PRG4 gene, MFAP5 gene, CRABP2 gene, HAS1 gene, CXCL1 gene, CXCL5 gene, CRLF1 gene, SPON1 gene, CXCL6 gene, S100A16 gene, DPT gene, MASP1 gene, CXCL8 gene, PTGIS gene, ACKR3 gene, GFPT2 gene, IL1RN gene, PDE4D gene, FBLN2 gene, CHI3L1 gene, NR4A1 gene, PID1 gene, ACKR4 gene, IL6 gene, ID3 gene, PTGFRN gene, EPB41L3 gene, PENK gene, ID1 gene, SFRP2 gene, HMCN1 gene, HS3ST3B1 gene, and PDPN gene, of which more preferable are one or more genes selected from the group consisting of PRG4 gene, MFAP5 gene, CRABP2 gene, HAS1 gene, CXCL1 gene, CXCL5 gene, CRLF1 gene, SPON1 gene, CXCL6 gene, S100A16 gene, DPT gene, MASP1 gene, CXCL8 gene, PTGIS gene, ACKR3 gene, GFPT2 gene, IL1RN gene, PDE4D gene, FBLN2 gene, CHI3L1 gene, NR4A1 gene, PID1 gene, ACKR4 gene, IL6 gene, ID3 gene, PTGFRN gene, EPB41L3 gene, PENK gene, ID1 gene, and SFRP2 gene,
of which further preferable are one or more genes selected from the group consisting of PRG4 gene, MFAP5 gene, CRABP2 gene, HAS1 gene, CXCL1 gene, CXCL5 gene, CRLF1 gene, SPON1 gene, CXCL6 gene, S100A16 gene, DPT gene, MASP1 gene, CXCL8 gene, PTGIS gene, ACKR3 gene, GFPT2 gene, IL1RN gene, PDE4D gene, FBLN2 gene, CHI3L1 gene, NR4A1 gene, PID1 gene, and ACKR4 gene,
of which still further preferable are one or more genes selected from the group consisting of PRG4 gene, MFAP5 gene, CRABP2 gene, HAS1 gene, CXCL1 gene, CXCL5 gene, CRLF1 gene, SPON1 gene, CXCL6 gene, S100A16 gene, DPT gene, MASP1 gene, CXCL8 gene, PTGIS gene, and ACKR3 gene.

When the expression of one or more genes selected from the gene group A or increased expression thereof is detected in step X, it can be determined that the cell population derived from an adipose tissue in step X comprises (or is likely to comprise) ASCs at high purity. This is because the genes in the gene group A have been confirmed to be not expressed or low expressed in a cell population derived from an adipose tissue, the cell population not comprising ASCs at high purity. On the other hand, the genes have been confirmed to be expressed or highly expressed in a cell population derived from an adipose tissue, the cell population comprising ASCs at high purity. Among the genes included in the gene group A, PTGIS or SFRP2 is not expressed in a cell population derived from an adipose tissue, the cell population not comprising ASCs at high purity. Therefore, when the expression of the gene is detected in a cell population, it can be determined that the cell population comprises (or is likely to comprise) ASCs at high purity.

The "increased expression of a gene" as used herein means that the expression level of the gene is increased as compared with the expression level of the gene in a cell population derived from an adipose tissue, the cell population not comprising ASCs at high purity. The increased expression of a gene may be confirmed by comparing the expression level of the gene in a cell population derived from an adipose tissue, the cell population not comprising ASCs at high purity, with the expression level of the gene in a cell population derived from an adipose tissue, the cell population comprising ASCs at high purity, in detecting the expression of the gene. In light of convenience, the increased expression may be confirmed by: using a numeric value obtained by measuring in advance the expression level of the gene in a cell population derived from an adipose tissue, the cell population not comprising ASCs at high purity; and comparing the expression level of the gene in ASCs with the numeric value.

The phrase "cell population derived from an adipose tissue comprises ASCs at high purity" as used herein means that: (X) the cell population is a cell population derived from an adipose tissue; and (Y) when the cell population is cultured in a basal medium for mesenchymal cell culture (preferably DMEM medium supplemented with bFGF at 20 ng/mL) for a given period (e.g., 3 to 14 days) and then the cell population thus cultured is cultured in an induction medium for differentiation into adipocytes (e.g., Adipocyte Differentiation Medium; manufactured by Cell Applications, Inc.) for a given period (e.g., 7 to 14 days), the number of mature adipocytes per 10000 cells in the cell population thus cultured can be 500 or more cells (preferably 1000 or more cells, more preferably 2000 or more cells, further preferably 3000 or more cells, more preferably 4000 or more cells, and further preferably 5000 or more cells).

Those skilled in the art can understand the nucleotide sequence of each gene included in the gene group A by utilizing a known sequence database such as NCBI. The nucleotide sequence of each gene listed in NCBI as of February 1, 2022 is preferable, and the nucleotide sequence of each human-derived gene listed in NCBI as of February 1, 2022 is more preferable. Tables 1 and 2 below illustrate accession No. of the nucleotide sequence of each human-derived gene.

**[Table 1]**

| Accession No. of nucleotide sequence of each gene |
|---|
| Human SFRP2 gene (cDNA encoding SFRP2); NM_003013.3 |
| Human PTGIS gene (cDNA encoding PTGIS); NM_000961.4 |
| Human PRG4 gene (cDNA encoding PRG4); XM_024448707.1 |
| Human MFAP5 gene (cDNA encoding MFAP5); NM_003480.4 |
| Human CRABP2 gene (cDNA encoding CRABP2); NM_001199723.2 |
| Human HAS1 gene (cDNA encoding HAS1); NM_001523.4 |
| Human CXCL1 gene (cDNA encoding. CXCL1); NM_001511.4 |
| Human CXCL5 gene (cDNA encoding CXCL5); NM_002994.5 |
| Human CRLF1 gene (cDNA encoding CRLF1); NM_004750.5 |
| Human SPON1 gene (cDNA encoding. SPON1); NM_006108.4 |
| Human CXCL6 gene (cDNA encoding CXCL6); NM_002993.4 |
| Human S100A16 gene (cDNA encoding. S100A16); NM_001317007.1 |
| Human DPT gene (cDNA encoding DPT); NM_001937.5 |
| Human MASP1 gene (cDNA encoding MASP1); NM_001879.6 |
| Human CXCL8 gene (cDNA encoding CXCL8); NM_000584.4 |
| Human ACKR3 gene (cDNA encoding ACKR3); NM_020311.3 |
| Human GFPT2 gene (cDNA encoding GFPT2); NM_005110.4 |
| Human IL1RN gene (cDNA encoding IL1RN); NM_173842.3 |
| Human PDE4D gene (cDNA encoding PDE4D); NM_001104631.2 |
| Human FBLN2 gene (cDNA encoding FBLN2); NM_001004019.2 |
| Human CHI3L1 gene (cDNA encoding CH13L1); NM_001276.4 |
| Human NR4A1 gene (cDNA encoding NR4A1); NM_173157.3 |
| Human PID1 gene (cDNA encoding PID1); NM_017933.5 |
| Human ACKR4 gene (cDNA encoding. ACKR4); NM_178445.2 |
| Human IL6 gene (cDNA encoding IL6); XM_005249745.5 |
| Human ID3 gene (cDNA encoding ID3); NM_002167.5 |
| Human PTGFRN gene (cDNA encoding PTGFRN); NM_020440.4 |
| Human EPB41L3 gene (cDNA encoding EPB41L3); NM_012307.5 |

**[Table 2]**

| Accession No. of nucleotide sequence of each gene |
|---|
| Human PENK gene (cDNA encoding PENK); NM_001135690.3 |
| Human ID1 gene (cDNA encoding ID1); NM_002165.4 |
| Human HMCN1 gene (cDNA encoding HMCN1); XM_011510038.3 |
| Human HS3ST3B1 gene (cDNA encoding HS3ST3B1); NM_006041.3 |
| Human PDPN gene (cDNA encoding PDPN); NM_006474.5 |
| Human HES1 gene (cDNA encoding HES1); NM_005524.4 |
| Human NDNF gene (cDNA encoding NDNF); XM_024454212.1 |
| Human KIAA1549L gene (cDNA encoding KIAA1549L); NM_012194.3 |
| Human LRRC15 gene (cDNA encoding LRRC15); NM_001135057.3 |
| Human DNASE1L3 gene (cDNA encoding DNASE1L3); NM_004944.4 |
| Human GAS7 gene (cDNA encoding GAS7); NM_003644.3 |
| Human FNDC1 gene (cDNA encoding FNDC1); NM_032532.3 |
| Human TWIST2 gene (cDNA encoding TWIST2); NM_001271893.4 |
| Human IRAK3 gene (cDNA encoding IRAK3); NM_007199.3 |
| Human SYNPO gene (cDNA encoding SYNPO); XM_006714755.3 |
| Human CBS gene (cDNA encoding CBS); NM_000071.3 |
| Human MFAP2 gene (cDNA encoding MFAP2); NM_017459.3 |
| Human BMP4 gene (cDNA encoding BMP4); NM_001347912.1 |
| Human MXRA5 gene (cDNA encoding MXRA5); NM_015419.4 |
| Human MMP12 gene (cDNA encoding MMP12); NM_002426.6 |
| Human CPXM1 gene (cDNA encoding CPXM1); NM_019609.5 |
| Human SGCD gene (cDNA encoding SGCD); NM_000337.6 |
| Human SIPA1L3 gene (cDNA encoding. SIPA1L3); NM_015073.3 |
| Human TBX5 gene (cDNA encoding TBX5); NM_000192.3 |
| Human PTPRN gene (cDNA encoding PTPRN); NM_002846.4 |
| Human JAM2 gene (cDNA encoding JAM2); NM_021219.4 |
| Human ID2 gene (cDNA encoding ID2); NM_002166.5 |
| Human ITPR3 gene (cDNA encoding ITPR3); NM_002224.4 |

The kit of the present invention is not particularly limited as long as the kit has a primer and/or a probe for detecting expression of mRNA or cDNA of one or more genes selected from the gene group A, or a labeled form thereof. Examples of the primer or the probe include the above nucleic acid fragment, and examples of the labeled form of the primer or the probe include the primer or the probe bound with a labeling substance. Examples of the labeling substance include a labeling substance based on one or more members selected from the group consisting of fluorescence, radiation, an enzyme, phosphorescence, chemiluminescence, and color.

The kit of the present invention may further have an enzyme or the like for use in gene microarray, PCR, or sequencing, in addition to the primer or the probe.

Further, one or more genes selected from the gene group A can be used as a biomarker for determining whether a cell population derived from a vertebrate animal adipose tissue comprises ASCs at high purity. More specifically, the biomarker of the present invention is a biomarker for determining whether a cell population derived from a vertebrate animal adipose tissue comprises ASCs at high purity, the biomarker consisting of one or more genes selected from the gene group A.

Hereinafter, the present invention will be described in more detail with reference to Examples. However, the present invention is not limited by these Examples.

### Example 1

### [Preparation of cell population derived from adipose tissue]

After isolation of subcutaneous adipose tissue pieces from a human, collagenase (collagenase type II; manufactured by Sigma-Aldrich) was added thereto and incubated at 37°C for 1 hour to obtain a cell suspension. As a result of centrifuging the cell suspension, mature adipocytes having a light specific gravity floated in a supernatant, and the other types of cells were precipitated as cell pellets. The cell pellets contain mesenchymal stem cell (including adipose progenitor cells)-like cells, stromal cells (stroma cells), vascular endothelial cells, smooth muscle cells, fibroblasts, and the like. The mesenchymal stem cell-like cells may be ASCs having differentiation potency into mature adipocytes or otherwise may be cells having no differentiation potency into mature adipocytes. In this point in time, the cells cannot be discriminated from each other even if morphologically observed under a microscope.

In subsequent experiments, the cells of the above cell pellets (that is, among the cells of the adipose tissue, a cell population of a stromal vascular fraction which is cells other than mature adipocytes) were used. The cells of the above cell pellets were cultured at 37°C under a CO₂ concentration condition of 5% for 3 to 7 days in a medium (DMEM medium supplemented with bFGF at 20 ng/mL) contained in a culture dish (culture corresponding to initial culture).

The cell population was prepared as 6 types from 6 different humans.

Total RNA was extracted from the cells of each cell population. Then, fluorescently labeled complementary RNA which was complementary to the total RNA was prepared. The above complementary RNA was hybridized with a DNA microarray (trade name: Clariom S; manufactured by Thermo Fisher Scientific Inc.) capable of detecting expression of 20800 types of genes. Then, the above DNA microarray was scanned, spot images were detected, and signal data was analyzed using software (trade name: Transcriptome Analysis Console (TAC) software; manufactured by Thermo Fisher Scientific Inc.).

Each cell population after the above initial culture was cultured at 37°C under a CO₂ concentration condition of 5% for 3 to 7 days in a medium (DMEM medium supplemented with bFGF at 20 ng/mL) contained in a culture dish (culture corresponding to expansion culture). Each cell population after the expansion culture was induced to differentiate into mature adipocytes by culturing the cells of the above cell pellets at 37°C under a CO₂ concentration condition of 5% for 10 days in Adipocyte Differentiation Medium (manufactured by Cell Applications, Inc.). The induction of differentiation yielded a cell population in which cells that differentiated into mature adipocytes were obtained at high purity (the number of mature adipocytes per 10000 cells in the cell population: approximately 5000 or more cells) and a cell population in which cells that differentiated into mature adipocytes were not obtained at high purity (the number of mature adipocytes per 10000 cells in the cell population: approximately 5000 or less cells). The ratios of expression levels in the cell population in which cells that differentiated into mature adipocytes were obtained at high purity (n = 4) to expression levels in the cell population in which cells that differentiated into mature adipocytes were not obtained at high purity (n = 2) were calculated as to the result of the DNA microarray described above (see Figure 1), and 56 types of genes (genes of the gene group A according to the present invention) having this ratio of 10 or more were selected. The ratios are shown in Tables 3 and 4.

**[Table 3]**

| Gene name | Expression ratio (fold) |
|---|---|
| PRG4 | 188.24 |
| MFAP5 | 95.32 |
| CRABP2 | 93.77 |
| HAS1 | 87.84 |
| CXCL1 | 78.66 |
| CXCL5 | 78.42 |
| CRLF1 | 63.78 |
| SPON1 | 61.23 |
| CXCL6 | 60.6 |
| S100A16 | 52.11 |
| DPT | 48.66 |
| MASP1 | 47.76 |
| CXCL8 | 47.04 |
| PTGIS | 43.58 |
| ACKR3 | 35.24 |
| GFPT2 | 31.21 |
| IL1RN | 30.64 |
| PDE4D | 30.2 |
| FBLN2 | 29.25 |
| CHI3L1 | 29.21 |
| NR4A1 | 28.83 |
| PID1 | 26.64 |
| ACKR4 | 26 |
| IL6 | 24.04 |
| ID3 | 22.81 |
| PTGFRN | 22.6 |
| EPB41L3 | 22.23 |
| PENK | 20.98 |

**[Table 4]**

| Gene name | Expression ratio (fold) |
|---|---|
| ID1 | 20.66 |
| SFRP2 | 19.02 |
| HMCN1 | 18.86 |
| HS3ST3B1 | 18.74 |
| PDPN | 18.07 |
| HES1 | 17.93 |
| NDNF | 16.91 |
| KIAA1549L | 16.82 |
| LRRC15 | 16.79 |
| DNASE1L3 | 15.82 |
| GAS7 | 15.36 |
| FNDC1 | 15.14 |
| TWIST2 | 15.12 |
| IRAK3 | 13.68 |
| SYNPO | 13.64 |
| CBS | 13.2 |
| MFAP2 | 12.97 |
| BMP4 | 12.68 |
| MXRA5 | 12.58 |
| MMP12 | 12.09 |
| CPXM1 | 12.08 |
| SGCD | 11.73 |
| SIPA1L3 | 11.53 |
| TBX5 | 11.39 |
| PTPRN | 11.21 |
| JAM2 | 10.98 |
| ID2 | 10.61 |
| ITPR3 | 10.36 |

The results of Tables 3 and 4 indicated that when expression of one or more genes selected from the gene group A or increased expression thereof is detected in a cell population after initial culture (primary culture), it can be determined that the cell population comprises ASCs at high purity.

### Industrial Applicability

The present invention can provide a method capable of more conveniently determining at an earlier stage whether a cell population collected from a vertebrate animal (donor, etc.) adipose tissue comprises mesenchymal stem cells (ASCs) at high purity, a kit for determining it, etc. The present invention is preferable because cost, time and labor, and wasted period can be reduced by determining earlier whether a cell population collected from a vertebrate animal (donor, etc.) adipose tissue comprises ASCs capable of producing ASCL at high purity in producing ASCL from the cell population using a cell line establishing method described in patent document 2.

## Claims

1. A method for determining whether a cell population derived from an adipose tissue collected from a vertebrate animal comprises mesenchymal stem cells (ASCs) at high purity, the method comprising step X of detecting expression of one or more genes selected from the following gene group A in the cell population:
gene group A:
SFRP2 (secreted frizzled-related protein 2) gene,
PTGIS (prostaglandin I2 (prostacyclin) synthase) gene,
PRG4 (proteoglycan 4) gene,
MFAP5 (microfibrillar associated protein 5) gene,
CRABP2 (cellular retinoic acid binding protein 2) gene,
HAS1 (hyaluronan synthase 1) gene,
CXCL1 (chemokine (C-X-C motif) ligand 1 (melanoma growth stimulating activity, alpha)) gene,
CXCL5 (chemokine (C-X-C motif) ligand 5) gene,
CRLF1 (cytokine receptor-like factor 1) gene,
SPON1 (spondin 1, extracellular matrix protein) gene,
CXCL6 (chemokine (C-X-C motif) ligand 6) gene,
S100A16 (S100 calcium binding protein A16) gene,
DPT (dermatopontin) gene,
MASP1 (mannan-binding lectin serine peptidase 1 (C4/C2 activating component of Ra-reactive factor)) gene,
CXCL8 (chemokine (C-X-C motif) ligand 8) gene,
ACKR3 (atypical chemokine receptor 3) gene,
GFPT2 (glutamine-fructose-6-phosphate transaminase 2) gene,
IL1RN (interleukin 1 receptor antagonist) gene,
PDE4D (phosphodiesterase 4D, cAMP-specific) gene,
FBLN2 (fibulin 2) gene,
CHI3L1 (chitinase 3-like 1 (cartilage glycoprotein-39) gene,
NR4A1 (nuclear receptor subfamily 4, group A, member 1) gene,
PID1 (phosphotyrosine interaction domain containing 1) gene,
ACKR4 (atypical chemokine receptor 4) gene,
IL6 (interleukin 6) gene,
ID3 (inhibitor of DNA binding 3, dominant negative helix-loop-helix protein) gene,
PTGFRN (prostaglandin F2 receptor inhibitor) gene,
EPB41L3 (erythrocyte membrane protein band 4.1-like 3) gene,
PENK (proenkephalin) gene,
ID1 (inhibitor of DNA binding 1, dominant negative helix-loop-helix protein) gene,
HMCN1 (hemicentin 1) gene,
HS3ST3B1 (heparan sulfate (glucosamine) 3-0-sulfotransferase 3B1) gene,
PDPN (podoplanin) gene,
HES1 (hes family bHLH transcription factor 1) gene,
NDNF (neuron-derived neurotrophic factor) gene,
KIAA1549L (KIAA1549-like) gene,
LRRC15 (leucine rich repeat containing 15) gene,
DNASE1L3 (deoxyribonuclease I-like 3) gene,
GAS7 (growth arrest-specific 7) gene,
FNDC1 (fibronectin type III domain containing 1) gene,
TWIST2 (twist family bHLH transcription factor 2) gene,
IRAK3 (interleukin 1 receptor associated kinase 3) gene,
SYNPO (synaptopodin) gene,
CBS (cystathionine-beta-synthase) gene,
MFAP2 (microfibrillar associated protein 2) gene,
BMP4 (bone morphogenetic protein 4) gene,
MXRA5 (matrix-remodelling associated 5) gene,
MMP12 (matrix metallopeptidase 12) gene,
CPXM1 (carboxypeptidase X (M14 family), member 1) gene,
SGCD (sarcoglycan delta) gene,
SIPA1L3 (signal-induced proliferation-associated 1 like 3) gene,
TBX5 (T-box 5) gene,
PTPRN (protein tyrosine phosphatase, receptor type, N) gene,
JAM2 (junctional adhesion molecule 2) gene,
ID2 (inhibitor of DNA binding 2, dominant negative helix-loop-helix protein) gene, and
ITPR3 (inositol 1,4,5-trisphosphate receptor, type 3) gene.

2. The method according to claim 1, wherein when the expression of one or more genes selected from the gene group A or increased expression thereof is detected in step X, it can be determined the cell population in step X comprises mesenchymal stem cells (ASCs) at high purity.

3. The method according to claim 1, wherein the cell population derived from an adipose tissue is a primary cultured cell population.

4. The method according to any one of claims 1 to 3, wherein the detection of the expression of one or more genes in step X is a detection using a gene microarray, PCR, or sequencing.

5. A kit for determining whether a cell population derived from a vertebrate animal adipose tissue comprises mesenchymal stem cells (ASCs) at high purity, the kit comprising a primer and/or a probe for detecting expression of mRNA or cDNA of one or more genes selected from the following gene group A, or a labeled form thereof:
gene group A:
SFRP2 (secreted frizzled-related protein 2) gene,
PTGIS (prostaglandin I2 (prostacyclin) synthase) gene,
PRG4 (proteoglycan 4) gene,
MFAP5 (microfibrillar associated protein 5) gene,
CRABP2 (cellular retinoic acid binding protein 2) gene,
HAS1 (hyaluronan synthase 1) gene,
CXCL1 (chemokine (C-X-C motif) ligand 1 (melanoma growth stimulating activity, alpha)) gene,
CXCL5 (chemokine (C-X-C motif) ligand 5) gene,
CRLF1 (cytokine receptor-like factor 1) gene,
SPON1 (spondin 1, extracellular matrix protein) gene,
CXCL6 (chemokine (C-X-C motif) ligand 6) gene,
S100A16 (S100 calcium binding protein A16) gene,
DPT (dermatopontin) gene,
MASP1 (mannan-binding lectin serine peptidase 1 (C4/C2 activating component of Ra-reactive factor)) gene,
CXCL8 (chemokine (C-X-C motif) ligand 8) gene,
ACKR3 (atypical chemokine receptor 3) gene,
GFPT2 (glutamine-fructose-6-phosphate transaminase 2) gene,
IL1RN (interleukin 1 receptor antagonist) gene,
PDE4D (phosphodiesterase 4D, cAMP-specific) gene,
FBLN2 (fibulin 2) gene,
CHI3L1 (chitinase 3-like 1 (cartilage glycoprotein-39) gene,
NR4A1 (nuclear receptor subfamily 4, group A, member 1) gene,
PID1 (phosphotyrosine interaction domain containing 1) gene,
ACKR4 (atypical chemokine receptor 4) gene,
IL6 (interleukin 6) gene,
ID3 (inhibitor of DNA binding 3, dominant negative helix-loop-helix protein) gene,
PTGFRN (prostaglandin F2 receptor inhibitor) gene,
EPB41L3 (erythrocyte membrane protein band 4.1-like 3) gene,
PENK (proenkephalin) gene,
ID1 (inhibitor of DNA binding 1, dominant negative helix-loop-helix protein) gene,
HMCN1 (hemicentin 1) gene,
HS3ST3B1 (heparan sulfate (glucosamine) 3-O-sulfotransferase 3B1) gene,
PDPN (podoplanin) gene,
HES1 (hes family bHLH transcription factor 1) gene,
NDNF (neuron-derived neurotrophic factor) gene,
KIAA1549L (KIAA1549-like) gene,
LRRC15 (leucine rich repeat containing 15) gene,
DNASE1L3 (deoxyribonuclease I-like 3) gene,
GAS7 (growth arrest-specific 7) gene,
FNDC1 (fibronectin type III domain containing 1) gene,
TWIST2 (twist family bHLH transcription factor 2) gene,
IRAK3 (interleukin 1 receptor associated kinase 3) gene,
SYNPO (synaptopodin) gene,
CBS (cystathionine-beta-synthase) gene,
MFAP2 (microfibrillar associated protein 2) gene,
BMP4 (bone morphogenetic protein 4) gene,
MXRA5 (matrix-remodelling associated 5) gene,
MMP12 (matrix metallopeptidase 12) gene,
CPXM1 (carboxypeptidase X (M14 family), member 1) gene,
SGCD (sarcoglycan delta) gene,
SIPA1L3 (signal-induced proliferation-associated 1 like 3) gene,
TBX5 (T-box 5) gene,
PTPRN (protein tyrosine phosphatase, receptor type, N) gene,
JAM2 (junctional adhesion molecule 2) gene,
ID2 (inhibitor of DNA binding 2, dominant negative helix-loop-helix protein) gene, and
ITPR3 (inositol 1,4,5-trisphosphate receptor, type 3) gene.

6. A biomarker for determining whether a cell population derived from a vertebrate animal adipose tissue comprises mesenchymal stem cells (ASCs) at high purity, the biomarker consisting of one or more genes selected from the following gene group A:
gene group A:
SFRP2 (secreted frizzled-related protein 2) gene,
PTGIS (prostaglandin I2 (prostacyclin) synthase) gene,
PRG4 (proteoglycan 4) gene,
MFAP5 (microfibrillar associated protein 5) gene,
CRABP2 (cellular retinoic acid binding protein 2) gene,
HAS1 (hyaluronan synthase 1) gene,
CXCL1 (chemokine (C-X-C motif) ligand 1 (melanoma growth stimulating activity, alpha)) gene,
CXCL5 (chemokine (C-X-C motif) ligand 5) gene,
CRLF1 (cytokine receptor-like factor 1) gene,
SPON1 (spondin 1, extracellular matrix protein) gene,
CXCL6 (chemokine (C-X-C motif) ligand 6) gene,
S100A16 (S100 calcium binding protein A16) gene,
DPT (dermatopontin) gene,
MASP1 (mannan-binding lectin serine peptidase 1 (C4/C2 activating component of Ra-reactive factor)) gene,
CXCL8 (chemokine (C-X-C motif) ligand 8) gene,
ACKR3 (atypical chemokine receptor 3) gene,
GFPT2 (glutamine-fructose-6-phosphate transaminase 2) gene,
IL1RN (interleukin 1 receptor antagonist) gene,
PDE4D (phosphodiesterase 4D, cAMP-specific) gene,
FBLN2 (fibulin 2) gene,
CHI3L1 (chitinase 3-like 1 (cartilage glycoprotein-39) gene,
NR4A1 (nuclear receptor subfamily 4, group A, member 1) gene,
PID1 (phosphotyrosine interaction domain containing 1) gene,
ACKR4 (atypical chemokine receptor 4) gene,
IL6 (interleukin 6) gene,
ID3 (inhibitor of DNA binding 3, dominant negative helix-loop-helix protein) gene,
PTGFRN (prostaglandin F2 receptor inhibitor) gene,
EPB41L3 (erythrocyte membrane protein band 4.1-like 3) gene,
PENK (proenkephalin) gene,
ID1 (inhibitor of DNA binding 1, dominant negative helix-loop-helix protein) gene,
HMCN1 (hemicentin 1) gene,
HS3ST3B1 (heparan sulfate (glucosamine) 3-0-sulfotransferase 3B1) gene,
PDPN (podoplanin) gene,
HES1 (hes family bHLH transcription factor 1) gene,
NDNF (neuron-derived neurotrophic factor) gene,
KIAA1549L (KIAA1549-like) gene,
LRRC15 (leucine rich repeat containing 15) gene,
DNASE1L3 (deoxyribonuclease I-like 3) gene,
GAS7 (growth arrest-specific 7) gene,
FNDC1 (fibronectin type III domain containing 1) gene,
TWIST2 (twist family bHLH transcription factor 2) gene,
IRAK3 (interleukin 1 receptor associated kinase 3) gene,
SYNPO (synaptopodin) gene,
CBS (cystathionine-beta-synthase) gene,
MFAP2 (microfibrillar associated protein 2) gene,
BMP4 (bone morphogenetic protein 4) gene,
MXRA5 (matrix-remodelling associated 5) gene,
MMP12 (matrix metallopeptidase 12) gene,
CPXM1 (carboxypeptidase X (M14 family), member 1) gene,
SGCD (sarcoglycan delta) gene,
SIPA1L3 (signal-induced proliferation-associated 1 like 3) gene,
TBX5 (T-box 5) gene,
PTPRN (protein tyrosine phosphatase, receptor type, N) gene,
JAM2 (junctional adhesion molecule 2) gene,
ID2 (inhibitor of DNA binding 2, dominant negative helix-loop-helix protein) gene, and
ITPR3 (inositol 1,4,5-trisphosphate receptor, type 3) gene.
